# NOUVEAU FASCICULE DE BREVET EUROPEEN

(11) **EP 1 307 491 B2**
(45) Date de publication et mention de la décision concernant l'opposition: **07.05.2014**
(45) Mention de la délivrance du brevet: 09.06.2010
(21) Numéro de dépôt: 01955436.9
(22) Date de dépôt: 18.07.2001
(51) Int. Cl.: C08B 37/10, A61K 31/727

(54) **MELANGES DE POLYSACCHARIDES DERIVES D'HEPARINE, LEUR PREPARATION ET LES COMPOSITIONS PHARMACEUTIQUES LES CONTENANT**
MISCHUNGEN VON POLYSACCHARIDEN AUS HEPARIN, DEREN HERSTELLUNG UND DIESE ENTHALTENDE PHARMAZEUTISCHE ZUBEREITUNGEN
HEPARIN-DERIVED POLYSACCHARIDE MIXTURES, PREPARATION METHOD AND PHARMACEUTICAL COMPOSITIONS CONTAINING SAME

(30) Priorité: 21.07.2000 FR 0009572
(43) Date de publication de la demande: 07.05.2003
(73) Titulaire: Aventis Pharma S.A., 92160 Antony (FR)
(72) Inventeur: DIAZ, Jacques, F-94170 LE PERREUX-SUR-MARNE (FR); PECQUET, Christelle, F-93130 NOISY LE SEC (FR); PERRIN, Elisabeth, F-27000 EVREUX (FR); VISKOV, Christian, F-91130 RIS ORANGIS (FR)
(86) Numéro de dépôt international: PCT/FR2001/002332
(87) Numéro de publication internationale: WO 2002/008295

(56) Documents cités:
- EP-A1- 0 027 089
- EP-A1- 0 040 144
- WO-A1-81/02737
- DATABASE CHEMABS [en ligne] CHEMICAL ABSTRACTS SERVICE, COLUMBUS, OHIO, US; VILA PAHI, F. JAVIER ET AL: "Preparation of saccharide oligomers by chemical depolymerization of heparin derivatives" retrieved from STN Database accession no. 125:303695 CA XP002165129 & ES 2 077 533 A1 (BIOIBERICA, S.A., SPAIN) 16 Novembre 1995

## Description

La présente invention concerne des mélanges de polysaccharides dérivés d'héparine, leur procédé de préparation et les compositions pharmaceutiques les contenant.

L'héparine est un mélange de mucopolysaccharides sulfatés d'origine animale, utilisée notamment pour ses propriétés anticoagulantes et antithrombotiques.

L'héparine présente cependant des inconvénients qui limitent les conditions de son utilisation. En particulier, son activité anticoagulante (activité anti-IIa) importante peut occasionner des hémorragies.

Des héparines de bas poids moléculaire obtenues par dépolymérisation basique d'esters d'héparine ont été proposées (EP40144); cependant ces produits ont encore une activité anti-IIa importante.

Le brevet EP 1 070 503 (Article 54(3) CBE) décrit des mélanges de polysaccharides présentant un poids moléculaire de 2000 à 4000 daltons, une activité anti-Xa comprise entre 100 et 150 UI/mg et un rapport anti-Xa/anti-IIa compris entre 15 et 50.

Ce document divulgue spécifiquement :
- une héparine de poids moléculaire moyen de 2777 daltons présentant une activité anti-Xa de 100 UI/mg et une activité anti-IIa de 4 UI/mg,
- une héparine de poids moléculaire moyen de 2161 daltons présentant une activité anti-Xa de 100 UI/mg et une activité anti-IIa de 2 UI/mg.

L'invention est relative à des mélanges de polysaccharides dérivés d'héparine possédant une activité plus sélective vis-à-vis du facteur X activé (facteur Xa) et du facteur II activé (facteur IIa) que l'héparine.

La présente invention a plus particulièrement pour objet les mélanges de polysaccharides sulfatés possédant la structure générale des polysaccharides constitutifs de l'héparine et présentant les caractéristiques suivantes :
- ils ont un poids moléculaire moyen de 1500 à 3000 daltons, une activité anti-Xa de 125 à 150 UI/mg, une activité anti-IIa de 0 à 10 UI/mg et un rapport activité anti-Xa/activité anti-IIa supérieur à 10,
- les polysaccharides constitutifs des mélanges contiennent 2 à 26 motifs saccharidiques et ont un motif acide glucuronique-4,5 insaturé 2-O-sulfate à l'une de leurs extrémités,
sous forme d'un sel de métal alcalin ou alcalinoterreux.

Comme sel de métal alcalin ou alcalinoterreux, sont préférés les sels de sodium, potassium, calcium et magnésium.

Le poids moléculaire moyen est déterminé par chromatographie liquide haute pression en utilisant deux colonnes en série par exemple celles commercialisées sous le nom de TSK G3000 XL et TSK G2000 XL. La détection est réalisée par réfractométrie. L'éluant utilisé est du nitrate de lithium et le débit est de 0,6 ml/min. Le système est calibré avec des standards préparés par fractionnement de l'enoxaparine (AVENTIS) par chromatographie sur gel d'agarose-polyacrylamide (IBF). Cette préparation est réalisée selon la technique décrite par Barrowcliffe et coll., Thromb. Res., 12, 27-36 (1977-78) ou D.A. Lane et coll., Thromb. Res., 12, 257-271 (1977-78). Les résultats sont calculés à l'aide du logiciel GPC6 (Perkin Elmer).

L'activité anti-Xa est mesurée par la méthode amidolytique sur un substrat chromogénique décrite par Teien et coll., Thromb. Res., 10, 399-410 (1977), avec comme étalon le premier étalon international des héparines de bas poids moléculaire.

L'activité anti-IIa est mesurée par la technique décrite par Anderson L.O. et coll., Thromb. Res., 15, 531-541 (1979), avec comme étalon le premier étalon international des héparines de bas poids moléculaire.

Tout particulièrement les mélanges tels que décrits plus haut présentent une activité anti Xa comprise entre 140 et 150 UI/mg et ont un poids moléculaire moyen compris entre 2000 et 3000 dalton

De préférence, les mélanges selon l'invention ont une activité anti-IIa de 0 à 5 UI/mg.

Encore plus préférentiellement, les mélanges présentent un rapport activité anti-Xa/activité anti-IIa supérieur à 25.

Les mélanges d'oligosaccharides selon l'invention peuvent être préparés par dépolymérisation d'un sel d'ammonium quaternaire de l'ester benzylique de l'héparine en milieu organique, au moyen d'une base organique forte de pka supérieur à 20 choisie parmi la 2-tert-butylimino-2-diéthylamino-1,3-diméthyl-perhydro-1,3,2-diazaphosphorine, les bases de la famille des guanidine et des phosphazènes, ou d'imidazolate de sodium, transformation du sel d'ammonium quaternaire de l'ester benzylique de l'héparine dépolymérisée en sel de sodium, saponification de l'ester et éventuellement purification.

Le sel d'ammonium quaternaire de l'ester benzylique de l'héparine est de préférence le sel de benzéthonium, de cétylpyridinium ou de cétyltriméthylammonium.

La dépolymérisation s'effectue généralement au sein d'un solvant organique inerte tel qu'un solvant chloré (dichlorométhane par exemple), le tétrahydrofurane, l'anisole, à une température de -20°C à 40°C.

Les bases de la famille des guanidines sont de préférence celles de formule: 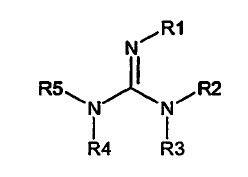 dans laquelle R₁ est hydrogène ou alkyle, R₂, R₃, R₄ et R₅ identiques ou différents représentent chacun un radical alkyle.

Plus particulièrement, R₁ est hydrogène et R₂, R₃, R₄ et R₅ sont des radicaux méthyle.

Les bases organiques fortes apparentées à la famille des phosphazènes sont définies par exemple selon R. Schwesinger et coll., Angew. Chem. Int. Ed. Engl. 26, 1167-1169 (1987), R. Schwesinger et coll., Angew. Chem. 105, 1420 (1993).

Parmi les bases de la famille des phosphazènes on utilise, de préférence, celles de formule : 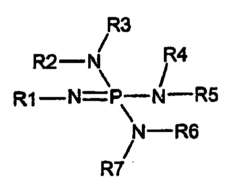 dans laquelle les radicaux R₁ à R₇ sont identiques ou différents et représentent des radicaux alkyle.

Dans les formules précédentes, les radicaux alkyle contiennent 1 à 6 atomes de carbone en chaîne droite ou ramifiée.

Avantageusement, le rapport en mole base organique forte de pka supérieur à 20 ou imidazolate de sodium/sel d'ammonium quaternaire de l'ester benzylique de l'héparine est compris entre 0,2 et 5 et, de préférence, entre 1 et 4.

Plus particulièrement, le taux d'estérification du sel d'ammonium quaternaire de l'ester benzylique de l'héparine est compris entre 50 et 100% et, de préférence, entre 70 et 90%. Ce taux d'estérification correspond au pourcentage molaire d'estérification des acides uroniques de l'héparine.

La transformation du sel d'ammonium quaternaire de l'ester benzylique de l'héparine dépolymérisée en sel de sodium est effectuée, généralement, par traitement du milieu réactionnel avec une solution alcoolique d'acétate de sodium et, de préférence, avec une solution à 10% d'acétate de sodium dans le méthanol (poids/volume), à une température comprise entre 15 et 25°C. L'équivalent en poids d'acétate ajouté est préferentiellement 3 fois supérieur à la masse de sel d'ammonium quaternaire de l'ester benzylique de l'héparine engagé dans la réaction de dépolymérisation.

La saponification s'effectue généralement au moyen d'un hydroxyde de métal alcalin tel que la soude, la potasse, l'hydroxyde de lithium, en milieu aqueux, à une température comprise entre 0 et 20°C et, e préférence, entre 0 et 10°C. On utilisera de façon générale de 1 à 5 équivalents molaires d'hydroxyde de métal alcalin. De préférence, la saponification sera réalisée en présence de 2 à 3 équivalents molaires d'hydroxyde de métal alcalin.

Le produit final peut éventuellement être purifié par toute méthode connue de purification des héparines dépolymérisées et notamment selon la méthode décrite dans le brevet EP 0037319. De préférence, on purifie au moyen de peroxyde d'hydrogène, en milieu aqueux, à une température de 10 à 50°C. De préférence, cette opération sera réalisée entre 20 et 40°C.

Le sel d'ammonium quaternaire de l'ester benzylique de l'héparine peut être préparé selon le schéma réactionnel suivant :
a) transformation de l'héparine sous forme de sel de sodium au moyen du chlorure de benzéthonium pour obtenir l'héparinate de benzéthonium,
b) estérification du sel de benzéthonium obtenu précédemment au moyen du chlorure de benzyle et traitement par une solution alcoolique d'acétate de sodium pour obtenir le sel de sodium de l'ester benzylique de l'héparine,
c) transalification du sel de sodium de l'ester benzylique de l'héparine en sel d'ammonium quaternaire et, de préférence en sel de benzéthonium, de cétylpyridinium ou de cétyltriméthylammonium,

La réaction de l'étape a) est réalisée en milieu aqueux, par action du chlorure de benzéthonium en excès, sur l'héparine sous forme de sel de sodium, à une température voisine de 15 à 25°C. De manière avantageuse, le rapport molaire chlorure de benzéthonium/héparine sous forme de sel de sodium est compris entre 2 et 3 et plus particulièrement 2,5.

L'héparine sous forme de sel de sodium de départ utilisée est de préférence une héparine de porc. Celle-ci peut être préalablement purifiée pour diminuer son taux de dermatane sulfate selon le procédé décrit dans le brevet FR2663639.

L'estérification de l'étape b) s'effectue de préférence au sein d'un solvant organique chloré (chloroforme, chlorure de méthylène par exemple), à une température comprise entre 25 et 45°C et, de préférence entre 30 et 40°C. L'ester sous forme de sel de sodium est ensuite récupéré par précipitation au moyen d'acétate de sodium à 10% en poids/volume dans un alcool tel que le méthanol. On emploie généralement 1 à 1,2 volume d'alcool par volume de milieu réactionnel. La quantité de chlorure de benzyle et le temps de réaction sont adaptés pour obtenir un taux d'estérification compris entre 50 et 100% et, de préférence, entre 70 et 90%. De façon préférentielle, on utilise 0,5 à 1,5 partie en poids du chlorure de benzyle pour 1 partie en poids du sel de benzéthonium de l'héparine. De même, de façon préférentielle, le temps de réaction sera compris entre 10 et 35 heures.

La transalification de l'étape c) s'effectue au moyen d'un chlorure d'ammonium quaternaire et, de préférence, au moyen de chlorure de benzéthonium, de chlorure de cétylpyridinium ou de chlorure de cétyltriméthylammonium, en milieu aqueux, à une température comprise entre 10 et 25°C. De manière avantageuse, le rapport en mole chlorure d'ammonium quaternaire/sel de sodium de l'ester benzylique de l'héparine est compris entre 2 et 3.

Les mélanges selon l'invention sous forme de sel de sodium, peuvent être transformés en un autre sel d'un métal alcalin ou alcalinoterreux. On passe éventuellement d'un sel à l'autre en utilisant la méthode décrite dans le brevet US4168377.

Les mélanges selon l'invention ne sont pas toxiques et peuvent ainsi être utilisés comme médicaments.

Les mélanges de la présente invention peuvent être utilisés comme agents antithrombotiques. En particulier, ils sont utiles pour la prévention des thromboses veineuses, des accidents thrombotiques artériels, notamment dans le cas d'infarctus du myocarde. Ils sont également utiles dans la prévention et le traitement de la prolifération des cellules musculaires lisses, l'angiogénèse, et comme agent neuroprotecteurs de l'athérosclérose et de l'artériosclérose.

La présente invention concerne également les compositions pharmaceutiques contenant comme principe actif un mélange de polysaccharides selon l'invention éventuellement en association avec un ou plusieurs excipients inertes.

Les compositions pharmaceutiques sont par exemple des solutions injectables par voie sous-cutanée ou intraveineuse. Elles sont également utiles par voie pulmonaire (inhalation).

La posologie peut varier en fonction de l'âge, du poids et de l'état de santé du patient. Pour un adulte, elle est en général comprise entre 20 et 100 mg par jour par voie intramusculaire ou sous-cutanée.

Les exemples suivants illustrent l'invention.

### EXEMPLE A : PREPARATION DU SEL DE BENZETHONIUM DE L'ESTER BENZYLIQUE DE L'HEPARINE

### Heparinate de benzéthonium

A une solution de 10 g d'héparine sous forme de sel de sodium dans 100 ml d'eau à une température voisine de 20°C, on ajoute une solution de 25 g de chlorure de benzéthonium dans 125 ml d'eau. Le produit est filtré, lavé à l'eau et séché.

### Ester benzylique d'héparine (sel de sodium)

A une solution de 20 g d'héparinate de benzéthonium dans 80 ml de chlorure de méthylène, on ajoute 16 ml de chlorure de benzyle. La solution est chauffée à une température de 30°C pendant 12 heures. On ajoute alors 108 ml d'une solution à 10% d'acétate de sodium dans le méthanol, filtre, lave au méthanol et sèche. On obtient ainsi 7,6 g d'ester benzylique d'héparine sous forme de sel de sodium dont le taux d'estérification est de 77%.

### Ester benzylique d'héparine (sel de benzéthonium)

Dans un erlenmeyer A de 2 litres, on introduit 36 g (0,0549 mole) d'ester benzylique d'héparine (sel de sodium) et 540 ml d'eau distillée. Après homogénéisation à une température d'environ 20°C, on obtient une solution jaune pâle. Sous agitation magnétique, on prépare dans un erlenmeyer B de 1 litre, une solution de 64,45 g (0,1438 mole) de chlorure de benzéthonium et de 450 ml d'eau. La solution de l'erlenmeyer B est coulée en environ 35 minutes dans la solution de l'ernenmeyer A sous agitation. On observe la formation d'un précipité blanc abondant. L'erlenmeyer B est rincé avec 200 ml d'eau distillée et l'eau de lavage est introduite dans l'erlenmeyer A. L'agitation est alors arrêtée et on laisse déposer la suspension pendant 12 heures. La partie limpide du surnageant est prélevée et écartée. Sur le précipité sédimenté (aspect de bouillie), on ajoute 560 ml d'eau et on agite pendant 20 minutes. On laisse re-sédimenter le précipité en 30 minutes environ. Le surnageant est prélevé et écarté (560 ml). Sur le précipité sédimenté, on renouvelle deux fois cette opération de lavage par environ 560 ml d'eau distillée. Dans la dernière opération de lavage, on laisse le précipité en suspension et on filtre sur Verre Fritté 3. Le gâteau est ensuite lavé par 4 fois 200 ml d'eau distillée. Le solide blanc humide est essoré puis séché sous pression réduite (2,7 kPa), à une température voisine de 60°C. Après 12 heures de séchage, on obtient 87,5 g d'ester benzylique d'héparine, sel de benzéthonium. Le rendement obtenu est de 94,9%.

### EXEMPLE 1 (Référence)

### Dépolymérisation et transformation en sel de sodium :

Dans un erlenmeyer A de 50 ml, on introduit 28 ml de dichlorométhane. Sous agitation, on charge lentement 4 g (0,00238 mole) d'ester benzylique d'héparine (taux d'estérification : 77%, sel de benzéthonium) obtenu comme décrit dans l'exemple A. Après dissolution complète, on ajoute 1,32 g (0,00948 mole) de 1,5,7-triazabicyclo[4.4.0]dec-5-ène. On agite à une température voisine de 20°C, pendant 3 heures et 30 minutes. Pendant ce temps, on prépare à 4°C dans un erlenmeyer B, une solution de 12 g d'acétate de sodium dans 120 ml de méthanol. Sous agitation magnétique, le mélange réactionnel de erlenmeyer A est coulé dans la solution méthanolique d'acétate de sodium. Un précipité jaune gélatineux quasi-translucide apparaît. L'agitation est alors arrêtée et on laisse décanter la suspension pendant une heure. La partie limpide du surnageant est prélevée et écartée (62 ml). Sur le précipité sédimenté (aspect de bouillie jaune), on ajoute 50 ml de méthanol et on agite pendant 20 minutes. On laisse re-sédimenter le précipité 30 minutes environ. Le surnageant est prélevé et écarté (49 ml). Sur le précipité sédimenté, on ajoute 50 ml de méthanol et on agite pendant 20 minutes. Le précipité en suspension est ensuite filtré sur Verre Fritté 4. Le gâteau jaune d'or obtenu est ensuite lavé par 2 fois 25 ml de méthanol. Le solide humide est essoré puis séché sous pression réduite (2,7 kPa), à une température voisine de 60°C. Après 12 heures de séchage, on obtient 1,21 g d'héparine dépolymérisée (ester de benzyle, sel de sodium). Le rendement obtenu est de 77,2%.

### Saponification :

Dans un erlenmeyer de 25 ml, on introduit 1,21 g (0,0018 mole) de l'héparine dépolymérisée (ester de benzyle, sel de sodium) obtenue précédemment et 11 ml d'eau. Sous agitation magnétique, on introduit 0,18 ml (0,0018 mole) de lessive de soude à 30%. Après addition, le mélange est refroidi à 4°C et agité pendant 2 heures. On ajoute 1,43 g de NaCl et on neutralise la solution par ajout d'HCl à 1mole/l (14 ml). Le mélange est transvasé dans un erlenmeyer de 100 ml et 52 ml de méthanol sont ajoutés. On observe la formation d'un précipité jaune. L'agitation est alors arrêtée et on laisse sédimenter la suspension pendant 12 heures à une température voisine de 20°C. Le surnageant est ensuite prélevé puis écarté (44 ml). Sur le précipité sédimenté (aspect de bouillie jaune), on ajoute 25 ml de méthanol et on agite pendant 20 minutes. On laisse re-sédimenter le précipité 30 minutes environ. Le surnageant est prélevé et écarté (21 ml). Sur le précipité sédimenté, on ajoute 25 ml de méthanol et on agite pendant 20 minutes. Le précipité en suspension est ensuite filtré sur Verre Fritté 3. Le gâteau jaune clair obtenu est ensuite lavé par 2 fois 10 ml de méthanol. Le solide humide est essoré puis séché sous pression réduite (2,7 kPa), à une température voisine de 60°C. Après 12 heures de séchage, on obtient 0,66 g d'héparine dépolymérisée brute (sel de sodium). Le rendement obtenu est de 60%.

### Purification :

Dans un erlenmeyer de 10 ml, on introduit 0,66 g d'héparine dépolymérisée brute obtenue précédemment et 5,9 ml d'eau distillée. Le mélange est porté à 40°C sous agitation magnétique. Par addition de soude à 0,1 mole/l, on porte le pH entre 9 et 10 et on ajoute 33 microlitres d'une solution aqueuse de peroxyde d'hydrogène à 30%. Après environ 2 heures d'agitation, on ajoute 0,65 g de chlorure de sodium. Le mélange est ensuite neutralisé par ajout d'HCl à 0,1mole/l. La solution est ensuite filtrée et tranvasée dans un erlenmeyer de 25 ml. On coule 23,3 ml de méthanol. On observe la formation d'un précipité blanc. L'agitation est alors arrêtée et on laisse sédimenter la suspension pendant 12 heures à une température voisine de 20°C. Le surnageant est ensuite prélevé puis écarté (5 ml). Sur le précipité sédimenté (aspect de bouillie), on ajoute 5 ml de méthanol et on agite pendant 20 minutes. On laisse re-sédimenter le précipité 30 minutes environ. Le surnageant est prélevé et écarté (5 ml). Sur le précipité sédimenté, on ajoute 5 ml de méthanol et on agite pendant 20 minutes. Le précipité en suspension est ensuite filtré sur Verre Fritté 3. Le gâteau blanc obtenu est ensuite lavé par 2 fois 5 ml de méthanol. Le solide humide est essoré puis séché sous pression réduite (2,7 kPa), à une température voisine de 60°C. Après 12 heures de séchage, on obtient 0,51 g d'un mélange purifié de polysaccharides (sel de sodium). Le rendement obtenu est de 77,2%.

Les caractéristiques de ce mélange sont les suivantes :
Poids moléculaire moyen : 1600 daltons
Activité anti-Xa : 94 UI/mg
Activité anti-IIa : < 0,1 UI/mg
Rapport activité anti-Xa/activité anti-IIa : > 100

### EXEMPLE 2

### Dépolymérisation et transformation en sel de sodium :

Dans un erlenmeyer A de 100 ml, on introduit 70 ml de dichlorométhane. Sous agitation, on charge lentement 10 g (0,00595 mole) d'ester benzylique d'héparine (taux d'estérification : 77%, sel de benzéthonium) obtenu comme décrit dans l'exemple A. Après dissolution complète, on ajoute 1,7 ml (0,00587 mole) de 2-tert-butylimino-2-diethylamino-1,3-dimethyl-perhydro-1,3,2-diazaphosphorine. A une température voisine de 20°C, on laisse la réaction se poursuivre pendant environ 3 heures et 30 minutes. Pendant ce temps, on prépare à 4°C dans un erlenmeyer B, une solution de 30 g d'acétate de sodium dans 300 ml de méthanol. Sous agitation magnétique, le mélange réactionnel de l'erlenmeyer A est coulé dans la solution méthanolique d'acétate de sodium. Un précipité jaune gélatineux quasi-translucide apparaît. L'agitation est alors arrêtée et on laisse décanter la suspension pendant une heure. La partie limpide du surnageant est prélevée et écartée (204 ml). Sur le précipité sédimenté (aspect de bouillie jaune), on ajoute 125 ml de méthanol et on agite pendant 20 minutes. On laisse re-sédimenter le précipité 30 minutes environ. Le surnageant est prélevé et écarté (162 ml). Sur le précipité sédimenté, on ajoute 125 ml de méthanol et on agite pendant 20 minutes. Le précipité gélatineux en suspension est ensuite filtré sur Verre Fritté 3. Le gâteau gélatineux jaune obtenu est ensuite lavé par 2 portions de 63 ml de méthanol. Le solide gélatineux est essoré puis séché sous pression réduite (2,7 kPa), à une température voisine de 60°C. Après 12 heures de séchage, on obtient 3,34 g d'héparine dépolymérisée (ester de benzyle, sel de sodium).
Le rendement obtenu est de 85,3%.

### Saponification :

On saponifie 1,67 g d'héparine dépolymérisée (ester de benzyle, sel de sodium) précédemment obtenue selon le procédé de saponification décrit dans l'exemple 1. On obtient 0,94 g d'une poudre jaune clair. Le rendement en héparine dépolymérisée brute (sel de sodium) est de 61%.

### Purification :

On purifie 0,94 g d'héparine dépolymérisée brute (sel de sodium) précédemment obtenue selon le procédé de purification décrit dans l'exemple 1. On obtient 0,71 g d'une poudre blanche. Le rendement est de 75,5%.

Le mélange purifié de polysaccharides (sel de sodium) obtenu présente les caractéristiques suivantes :
Poids moléculaire moyen : 2500 Daltons
Activité anti-Xa : 146,6 UI/mg
Activité anti-IIa : 2,15 UI/mg
Rapport activité anti-Xa/activité anti-IIa : 68

### EXEMPLE 3 (Référence)

### Dépolymérisation et transformation en sel de sodium :

Dans un erlenmeyer A de 50 ml, on introduit 28 ml de dichlorométhane. Sous agitation, on charge lentement 4 g (0,00238 mole) d'ester benzylique d'héparine (taux d'estérification : 77%, sel de benzéthonium) obtenu selon l'exemple A. Après dissolution complète et refroidissement à 2°C, on ajoute 0,333 g (0,00239 mole) de 1,5,7-triazabicyclo[4.4.0]dec-5-ène. A une température voisine de 20°C, on laisse la réaction se poursuivre pendant environ 3 heures et 30 minutes. Pendant ce temps, on prépare à 4°C dans un erlenmeyer B, une solution de 12 g d'acétate de sodium dans 120 ml de méthanol. Sous agitation magnétique, le mélange réactionnel de l'erlenmeyer A est coulé dans la solution méthanolique d'acétate de sodium. Un précipité jaune apparaît. L'agitation est alors arrêtée et on laisse décanter la suspension pendant une heure. La partie limpide du surnageant est prélevée et écartée (90 ml). Sur le précipité sédimenté (aspect de bouillie jaune), on ajoute 50 ml de méthanol et on agite pendant 20 minutes. On laisse re-sédimenter le précipité 30 minutes environ. Le surnageant est prélevé et écarté (61 ml). Sur le précipité sédimenté, on ajoute 50 ml de méthanol et on agite pendant 20 minutes. Le précipité en suspension est ensuite filtré sur Verre Fritté 4. Le gâteau obtenu est ensuite lavé par 2 fois 25 ml de méthanol. Le solide humide est essoré puis séché sous pression réduite (2,7 kPa), à une température voisine de 60°C. Après 12 heures de séchage, on obtient 1,19 g d'héparine dépolymérisée (ester de benzyle, sel de sodium). Le solide est jaune intense. Le rendement obtenu est de 75,9%.

### Saponification :

On saponifie 1,19 g d'héparine dépolymérisée (ester de benzyle, sel de sodium) précédemment obtenue selon le procédé de saponification décrit dans l'exemple 1. On obtient 0,78 g d'une poudre jaune clair. Le rendement en héparine dépolymérisée brute (sel de sodium) est de 71,5%.

### Purification :

On purifie 0,78 g d'héparine dépolymérisée brute (sel de sodium) précédemment obtenue selon la méthode de purification décrite dans l'exemple 1. On obtient 0,58 g d'une poudre blanche. Le rendement est de 72,5%.

Le mélange purifié de polysaccharides (sel de sodium) obtenu présente les caractéristiques suivantes :
Poids moléculaire moyen : 2700 Daltons
Activité anti-Xa : 100,1 UI/mg
Activité anti-IIa : 3,3 UI/mg
Rapport activité anti-Xa/activité anti-IIa : 27,3

### EXEMPLE 4

### Dépolymérisation et transformation en sel de sodium :

Dans un erlenmeyer A de 50 ml, on introduit 28 ml de dichlorométhane. Sous agitation, on charge lentement 4 g (0,00238 mole) d'ester benzylique de l'héparine (taux d'estérification : 77%, sel de benzéthonium) obtenu comme décrit dans l'exemple A. Après dissolution complète et à 2°C, on ajoute 0,6 ml (0,00222 mole) de 2-tert-butylimino-tris(diméthylamino)phosphorane. A une température voisine de 0°C, on laisse la réaction se poursuivre pendant environ 3 heures et 30 minutes. Pendant ce temps, on prépare à 4°C dans un erlenmeyer B, une solution de 12 g d'acétate de sodium dans 120 ml de méthanol. Sous agitation magnétique, le mélange réactionnel de l'erlenmeyer A est coulé dans la solution méthanolique d'acétate de sodium. Un précipité jaune gélatineux quasi-translucide apparaît. L'agitation est alors arrêtée et on laisse décanter la suspension pendant une heure. La partie limpide du surnageant est prélevée et écartée (108 ml). Sur le précipité sédimenté (aspect de bouillie jaunâtre), on ajoute 50 ml de méthanol et on agite pendant 20 minutes. On laisse re-sédimenter le précipité 30 minutes environ. Le surnageant est prélevé et écarté (60 ml). Sur le précipité sédimenté, on ajoute 50 ml de méthanol et on agite pendant 20 minutes. Le précipité blanc jaunâtre en suspension est ensuite filtré sur Verre Fritté 4. Le gâteau obtenu est ensuite lavé par 2 fois 25 ml de méthanol. Le solide est essoré puis séché sous pression réduite (2,7 kPa), à une température voisine de 60°C. Après 12 heures de séchage, on obtient 1,22 g d'héparine dépolymérisée (ester de benzyle, sel de sodium). Le rendement obtenu est de 77,8 %.

### Saponification :

On saponifie 1,22 g d'héparine dépolymérisée (ester de benzyle, sel de sodium) précédemment obtenue selon le protocole de saponification décrit dans l'exemple 1.

On obtient 0,69 g d'une poudre jaune très clair. Le rendement en héparine dépolymérisée brute (sel de sodium) est de 61,6%.

### Purification :

On purifie 0,69 g d'héparine dépolymérisée brute (sel de sodium) précédemment obtenue selon le protocole de purification décrit dans l'exemple 1. On obtient 0,67 g d'une poudre blanche. Le rendement est de 97,1 %.

Le mélange purifié de polysaccharides (sel de sodium) obtenu présente les caractéristiques suivantes :
Poids moléculaire moyen : 2900 Daltons
Activité anti-Xa : 145,2 UI/mg
Activité anti-IIa : 4,5 UI/mg
Rapport activité anti-Xa/activité anti-IIa : 32,6

### EXEMPLE 5

### Dépolymérisation et transformation en sel de sodium :

Dans un ballon A de 50 ml, on introduit 28 ml de dichlorométhane. Sous agitation, on charge lentement 4 g (0,00238 mole) d'héparinate de benzyle (taux d'estérification : 77%, sel de benzéthonium). Après dissolution complète et à 40°C, on ajoute 0,95 g (0,00949 mole) d'imidazolate de sodium. Au reflux du dichlorométhane, on laisse la réaction se poursuivre pendant environ 4 heures. Pendant ce temps, on prépare à 4°C dans un erlenmeyer B, une solution de 12 g d'acétate de sodium dans 120 ml de méthanol. Sous agitation magnétique, le mélange réactionnel de l'erlenmeyer A est coulé dans la solution méthanolique d'acétate de sodium. Un précipité jaune gélatineux quasi-translucide apparaît. L'agitation est alors arrêtée et on laisse décanter la suspension pendant une heure. La partie limpide du surnageant orange est prélevée et écartée (88 ml). Sur le précipité sédimenté (aspect de bouillie orange), on ajoute 50 ml de méthanol et on agite pendant 20 minutes. On laisse re-sédimenter le précipité 30 minutes environ. Le surnageant est prélevé et écarté (51 ml). Sur le précipité sédimenté, on ajoute 50 ml de méthanol et on agite pendant 20 minutes. Le précipité orange en suspension est ensuite filtré sur Verre Fritté 4. Le gâteau obtenu est ensuite lavé par 2 fois 25 ml de méthanol. Le solide est essoré puis séché sous pression réduite (2,7 kPa), à une température voisine de 60°C. Après 12 heures de séchage, on obtient 1,34 g d'héparine dépolymérisée (ester de benzyle, sel de sodium).
Le rendement obtenu est de 76,6%.

### Saponification :

On saponifie 1,2 g d'héparine dépolymérisée (ester de benzyle, sel de sodium) précédemment obtenue selon le protocole de saponification décrit dans l'exemple 1.

On obtient 0,63 g d'une poudre beige. Le rendement en héparine dépolymérisée brute (sel de sodium) est de 52,5%.

### Purification :

On purifie 0,63 g d'héparine dépolymérisée brute (sel de sodium) précédemment obtenue selon la méthode de purification décrite dans l'exemple 1. On obtient 0,42 g d'une poudre blanc beige. Le rendement est de 66,7%.

Le mélange purifié de polysaccharides (sel de sodium) obtenu présente les caractéristiques suivantes :
Poids moléculaire moyen : 2250 Daltons
Activité anti-Xa : 134,5 UI/mg
Activité anti-IIa : 1,5 UI/mg
Rapport activité anti-Xa/activité anti-IIa : 90,5

### EXEMPLE 6 (Référence)

### Dépolymérisation et transformation en sel de sodium :

Dans un erlenmeyer A de 50 ml, on introduit 28 ml de dichlorométhane. Sous agitation, on charge lentement 4 g (0,00238 mole) d'ester benzylique d'héparine (taux d'estérification : 77%, sel de benzéthonium) obtenu comme décrit dans l'exemple A. Après dissolution complète, on ajoute 1,33 g (0,00956 mole) de 1,5,7-triazabicyclo[4.4.0]dec-5ène. On agite à une température voisine de 20°C, pendant 3 heures et 30 minutes. Pendant ce temps, on prépare à 4°C dans un erlenmeyer B, une solution de 12 g d'acétate de sodium dans 120 ml de méthanol. Sous agitation magnétique, le mélange réactionnel de l'erlenmeyer A est coulé dans la solution méthanolique d'acétate de sodium. Un précipité jaune gélatineux quasi-translucide apparaît. L'agitation est alors arrêtée et on laisse décanter la suspension pendant une heure. La partie limpide du surnageant est prélevée et écartée (56 ml). Sur le précipité sédimenté (aspect de bouillie jaune), on ajoute 60 ml de méthanol et on agite pendant 20 minutes. On laisse re-sédimenter le précipité 30 minutes environ. Le surnageant est prélevé et écarté (70 ml). Sur le précipité sédimenté, on ajoute 50 ml de méthanol et on agite pendant 15 minutes. Le précipité en suspension est ensuite filtré sur Verre Fritté 4. Le gâteau jaune d'or obtenu est ensuite lavé par 2 fois 50 ml de méthanol. Le solide humide est essoré puis séché sous pression réduite (2,7 kPa), à une température voisine de 60°C. Après 12 heures de séchage, on obtient 0,92 g d'héparine dépolymérisée (ester de benzyle, sel de sodium). Le rendement obtenu est de 64 %.

### Saponification :

Dans un erlenmeyer de 25 ml, on introduit 0,92 g (0,0014 mole) de l'héparine dépolymérisée (ester de benzyle, sel de sodium) obtenue précédemment et 17,5 ml d'eau. Sous agitation magnétique, on introduit 0,38 ml (0,00379 mole) de lessive de soude à 30%. Après addition, le mélange est maintenu à une température voisine de 20°C et agité pendant 5 heures. On ajoute 1,8 g de NaCl et on neutralise la solution par ajout d'HCl à concentré (volume final de la solution environ 18 ml). Le mélange est transvasé dans un erlenmeyer de 100 ml et 46 ml de méthanol sont ajoutés. On observe la formation d'un précipité jaune. L'agitation est alors arrêtée et on laisse sédimenter la suspension pendant 12 heures à une température voisine de 20°C. Le surnageant est ensuite prélevé puis écarté (52 ml). Sur le précipité sédimenté (aspect de bouillie jaune), on ajoute 25 ml de méthanol et on agite pendant 20 minutes. On laisse re-sédimenter le précipité 1 heure environ. Le surnageant est prélevé et écarté (27 ml). Sur le précipité sédimenté, on ajoute 25 ml de méthanol et on agite pendant 1 heure environ. Le précipité en suspension est ensuite filtré sur Verre Fritté 4. Le gâteau obtenu est ensuite lavé par 2 fois 10 ml de méthanol. Le solide humide est essoré puis séché sous pression réduite (2,7 kPa), à une température voisine de 60°C. Après 3 heures de séchage, on obtient 0,42 g d'héparine dépolymérisée brute (sel de sodium). Le rendement obtenu est de 45,6 %.

### Purification :

Dans un erlenmeyer de 10 ml, on introduit 0,42 g d'héparine dépolymérisée brute obtenue précédemment et 3,8 ml d'eau distillée. Le mélange est porté à 38°C sous agitation magnétique. Par addition de soude à 0,1 mole/l, on porte le pH entre 9 et 10 et on ajoute 25 microlitres d'une solution aqueuse de peroxyde d'hydrogène à 30%. Après environ 2 heures 30 minutes d'agitation, on ajoute 0,5 g de chlorure de sodium. Le mélange est ensuite neutralisé par ajout d'HCl à 0,1mole/l. La solution est ensuite filtrée et tranvasée dans un erlenmeyer de 25 ml. On coule 11,3 ml de méthanol. On observe la formation d'un précipité blanc. L'agitation est alors arrêtée et on laisse sédimenter la suspension pendant 12 heures à une température voisine de 20°C. Le surnageant est ensuite prélevé puis écarté (9,8 ml). Sur le précipité sédimenté (aspect de bouillie), on ajoute 5 ml de méthanol et on agite pendant 15 minutes. On laisse re-sédimenter le précipité 3 heures environ. Le surnageant est prélevé et écarté (6,2 ml). Sur le précipité sédimenté, on ajoute 5 ml de méthanol et on agite pendant 20 minutes. Le précipité en suspension est ensuite filtré sur Verre Fritté 3. Le gâteau blanc obtenu est ensuite lavé par 2 fois 5 ml de méthanol. Le solide humide est essoré puis séché sous pression réduite (2,7 kPa), à une température voisine de 60°C. Après environ 2 heures 20 minutes de séchage, on obtient 0,39 g d'héparine dépolymérisée pure (sel de sodium). Le rendement obtenu est de 92,8%.

Les caractéristiques de l'héparine dépolymérisée ainsi obtenue sont les suivantes :
Poids moléculaire moyen : 1950 daltons
Activité anti-Xa : 115,6 UI/mg
Activité anti-IIa : < 2 UI/mg
Rapport activité anti-Xa/activité anti-IIa : > 57

### EXEMPLE 7

### Dépolymérisation et transformation en sel de sodium :

Dans un réacteur A de 400 ml, on introduit 140 ml de dichlorométhane. Sous agitation, on charge lentement 20 g (0,0119 mole) d'ester benzylique d'héparine (taux d'estérification : 77%, sel de benzéthonium) obtenu comme décrit dans l'exemple A. Après dissolution complète, on mesure la teneur en eau du milieu réactionnel par la méthode de Karl Fisher. La valeur obtenue est de 0,1% d'eau. On ajoute alors 3,5 ml (0,0121 mole) de 2-tert-butylimino-2-diethylamino-1,3-dimethyl-perhydro-1,3,2-diazaphosphorine. A une température voisine de 25°C, on laisse la réaction se poursuivre pendant environ 24 heures. Pendant ce temps, on prépare à 4°C dans un erlenmeyer B, une solution de 30 g d'acétate de sodium dans 300 ml de méthanol. Sous agitation magnétique, la moitié du mélange réactionnel du réacteur A est coulé dans la solution méthanolique d'acétate de sodium. Un précipité jaune gélatineux quasi-translucide apparaît. L'agitation est maintenue pendant une heure et on laisse décanter la suspension pendant environ 12 heures à 4°C. La partie limpide du surnageant est prélevée et écartée (220 ml). Sur le précipité sédimenté (aspect de bouillie jaune), on ajoute 220 ml de méthanol et on agite pendant 50 minutes. On laisse re-sédimenter le précipité 40 minutes environ. Le surnageant est prélevé et écarté (204 ml). Sur le précipité sédimenté, on ajoute 204 ml de méthanol et on agite pendant 40 minutes. Le précipité gélatineux en suspension est ensuite filtré sur Verre Fritté 3. Le gâteau gélatineux jaune obtenu est ensuite lavé par 2 portions de 100 ml de méthanol. Le solide gélatineux est essoré puis séché sous pression réduite (2,7 kPa), à une température voisine de 60°C. Après environ 12 heures de séchage, on obtient 2,6 g d'héparine dépolymérisée (ester de benzyle, sel de sodium). Le rendement obtenu est de 70,6 % (calculé sur la base de la moitié du milieu réactionnel traité).

### Saponification :

On saponifie 2,6 g d'héparine dépolymérisée (ester de benzyle, sel de sodium) précédemment obtenue selon le procédé de saponification décrit dans l'exemple 1. On obtient 1,48 g d'une poudre jaune clair. Le rendement en héparine dépolymérisée brute (sel de sodium) est de 62,9%.

### Purification :

Dans un erlenmeyer de 50 ml, on introduit 1,48 g d'héparine dépolymérisée brute obtenue précédemment et 15 ml d'eau distillée. Le mélange est porté à 40°C sous agitation magnétique. Par addition de soude à 1 mole/l, on porte le pH entre 9 et 10. La solution est filtrée sur une membrane fitrante de porosité 1 µm. On ajoute ensuite 76 microlitres d'une solution aqueuse de peroxyde d'hydrogène à 30%. Après environ 2 heures d'agitation, on ajoute 1,5 g de chlorure de sodium. Le mélange est ensuite neutralisé par ajout d'HCl à 1mole/l. La solution est filtrée sur une membrane fitrante de porosité 1 µm. On coule 38 ml de méthanol. On observe la formation d'un précipité blanc. L'agitation est alors arrêtée et on laisse sédimenter la suspension pendant 1 heures à une température voisine de 20°C. Le surnageant est ensuite prélevé puis écarté (37 ml). Sur le précipité, on ajoute 37 ml de méthanol et on agite pendant 45 minutes. On laisse re-sédimenter le précipité 45 minutes environ. Le surnageant est prélevé et écarté (34 ml). Sur le précipité sédimenté, on ajoute 34 ml de méthanol et on agite pendant 15 minutes. Le précipité en suspension est ensuite filtré sur Verre Fritté 3. Le gâteau blanc obtenu est ensuite lavé par 2 fois 25 ml de méthanol. Le solide humide est essoré puis séché sous pression réduite (2,7 kPa), à une température voisine de 60°C. Après 12 heures de séchage, on obtient 1,29 g d'héparine dépolymérisée pure (sel de sodium). Le rendement obtenu est de 87,2%.

L'héparine dépolymérisée purifiée (sel de sodium) obtenue présente les caractéristiques suivantes :
Poids moléculaire moyen : 2250 Daltons
Activité anti-Xa : 149,6 UI/mg
Activité anti-IIa : 0,85 UI/mg
Rapport activité anti-Xa/activité anti-IIa : 176

## Revendications

1. Mélanges de polysaccharides sulfatés possédant la structure générale des polysaccharides constitutifs de l'héparine et présentant les caractéristiques suivantes :
- ils ont un poids moléculaire moyen de 1500 à 3000 Daltons, une activité anti-Xa de 125 à 150 UI/mg, une activité anti-IIa de 0 à 10 UI/mg et un rapport activité anti-Xa/activité anti-IIa supérieur à 10,
- les polysaccharides constitutifs des mélanges contiennent 2 à 26 motifs saccharidiques et ont un motif acide glucuronique-4,5 insaturé 2-O-sulfate à l'une de leurs extrémités,
sous forme d'un sel de métal alcalin ou alcalinoterreux.

2. Mélanges selon la revendication 1 **caractérisé en ce que** l'activité anti-Xa est comprise entre 140 et 150 UI/mg et le poids moléculaire moyen est compris entre 2000 et 3000 Daltons.

3. Mélanges selon la revendication 1 ou 2 sous forme de sel de sodium, de potassium, de calcium ou de magnésium.

4. Mélanges selon l'une quelconque des revendications 1 à 3 ayant une activité anti-IIa de 0 à 5 UI/mg.

5. Mélanges selon l'une quelconque des revendications 1 à 4 présentant un rapport activité anti-Xa/activité anti-IIa supérieur à 25.

6. Procédé de préparation des mélanges de la revendication 1, **caractérisé en ce que** l'on :
- dépolymérise un sel d'ammonium quaternaire de l'ester benzylique de l'héparine en milieu organique, au moyen :
• d'une base organique forte de pka supérieur à 20 choisie parmi la 2-tert-butylimino-2-diéthylamino-1,3-diméthylperhydro-1,2,3-diazaphosphorine, les bases de la famille de la guanidine et des phosphazènes,
• ou d'imidazolate de sodium,
- transforme le sel d'ammonium quaternaire de l'ester benzylique de l'héparine dépolymérisée en sel de sodium,
- saponifie l'ester et éventuellement purifie le produit.

7. Procédé selon la revendication 6 pour lequel le sel d'ammonium quaternaire de l'ester benzylique de l'héparine est le sel de benzéthonium, de cétylpyridinium ou de cétyltriméthylammonium.

8. Procédé selon la revendication 6 pour lequel les bases de la famille des guanidines sont celles de formule : 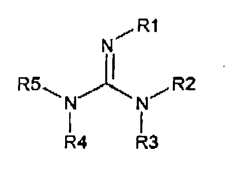 dans laquelle R₁ est hydrogène ou alkyle, R₂, R₃, R₄ et R₅ identiques ou différents représentent chacun un radical alkyle, les radicaux alkyle ayant 1 à 6 atomes de carbone en chaîne droite ou ramifiée.

9. Procédé selon la revendication 8 pour lequel R₁ est hydrogène et R₂, R₃, R₄ et R₅ sont des radicaux méthyle.

10. Procédé selon la revendication 6 pour lequel les bases de la famille des phosphazènes sont celles de formule : 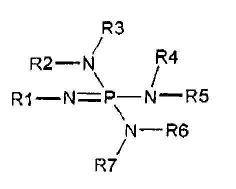 dans laquelle les radicaux R₁ à R₇ sont identiques ou différents et représentent des radicaux alkyle contenant 1 à 6 atomes de carbone, en chaîne droite ou ramifiée.

11. Procédé selon l'une des revendications 6 à 10 pour lequel le rapport en mole base organique forte de pka supérieur à 20 ou imidazolate de sodium/sel d'ammonium quaternaire de l'ester benzylique de l'héparine est compris entre 0,2 et 5.

12. Procédé selon l'une des revendications 6 à 11 pour lequel le sel d'ammonium quaternaire de l'ester benzylique de l'héparine a un taux d'esterification compris entre 50 et 100%.

13. Procédé selon l'une des revendications 6 à 12 pour lequel la transformation du sel d'ammonium quaternaire de l'ester benzylique de l'héparine dépolymérisée en sel de sodium est effectuée par traitement du milieu réactionnel avec une solution alcoolique d'acétate de sodium.

14. Procédé selon l'une des revendications 6 à 13 pour lequel la saponification s'effectue au moyen d'un hydroxyde de métal alcalin.

15. Procédé selon l'une des revendication 6 à 14 pour lequel la purification s'effectue au moyen de peroxyde d'hydrogène.

16. Mélanges tels que définis à l'une quelconque des revendications 1 à 5 à titre de médicaments.

17. Mélanges tels que définis à l'une quelconque des revendications 1 à 5 à titre de médicaments antithrombotiques.

18. Compositions pharmaceutiques comprenant en tant que principe actif un mélange selon l'une des revendications 1 à 5.

## Patentansprüche

1. Gemische von sulfatierten Polysacchariden mit der allgemeinen Struktur der Polysaccharide, aus denen Heparin aufgebaut ist, und den folgenden Eigenschaften:
- sie haben ein mittleres Molekulargewicht von 1500 bis 3000 Dalton, eine Anti-Xa-Aktivität von 125 bis 150 IE/mg, eine Anti-IIa-Aktivität von 0 bis 10 IE/mg und ein Verhältnis von Anti-Xa-Aktivität zu Anti-IIa-Aktivität von mehr als 10,
- die Polysaccharide, aus denen das Gemisch besteht, enthalten 2 bis 26 Saccharid-Einheiten und weisen an einem ihrer Enden eine 4,5-ungesättigte Glucuronsäure-2-O-sulfat-Einheit auf,
in Form eines Alkalimetall- oder Erdalkalimetallsalzes.

2. Gemische nach Anspruch 1, **dadurch gekennzeichnet, daß** die Anti-Xa-Aktivität zwischen 140 und 150 IE/mg liegt und das mittlere Molekulargewicht zwischen 2000 und 3000 Dalton liegt.

3. Gemische nach Anspruch 1 oder 2 in Form eines Natrium-, Kalium-, Calcium- oder Magnesiumsalzes.

4. Gemische nach einem der Ansprüche 1 bis 3 mit einer Anti-IIa-Aktivität von 0 bis 5 IE/mg.

5. Gemische nach einem der Ansprüche 1 bis 4 mit einem Verhältnis von Anti-Xa-Aktivität zu Anti-IIa-Aktivität von mehr als 25.

6. Verfahren zur Herstellung der Gemische nach Anspruch 1, **dadurch gekennzeichnet, daß** man:
- ein quartäres Ammoniumsalz von Heparinbenzylester in organischem Medium mit Hilfe
• einer starken organischen Base mit einem pKa-Wert von mehr als 20, die unter 2-tert.-Butyl-imino-2-diethylamino-1,3-dimethylperhydro-1,3,2-diazaphosphorin, Basen aus der Familie von Guanidin und Phosphazenen ausgewählt wird,
• oder Natriumimidazolat
depolymerisiert,
- das depolymerisierte quartäre Ammoniumsalz von Heparinbenzylester in ein Natriumsalz umwandelt,
- den Ester verseift und das Produkt gegebenenfalls reinigt.

7. Verfahren nach Anspruch 6, für das es sich bei dem quartären Ammoniumsalz von Heparinbenzylester um das Benzethonium-, Cetylpyridinium- oder Cetyltrimethylammoniumsalz handelt.

8. Verfahren nach Anspruch 6, für das die Basen aus der Familie der Guanidine diejenigen der Formel: 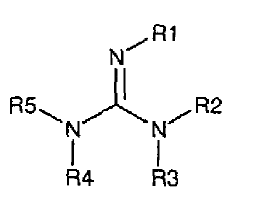 worin R₁ für Wasserstoff oder Alkyl steht und R₂, R₃, R₄ und R₅ gleich oder verschieden sind und jeweils für einen Alkylrest stehen, wobei die Alkylreste 1 bis 6 Kohlenstoffatome in gerader oder verzweigter Kette aufweisen, sind.

9. Verfahren nach Anspruch 8, für das R₁ für Wasserstoff steht und R₂, R₃, R₄ und R₅ für Methylreste stehen.

10. Verfahren nach Anspruch 6, für das die Basen aus der Familie der Phosphazene diejenigen der Formel: 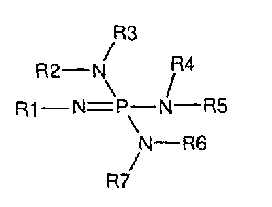 worin die Reste R₁ bis R₇ gleich oder verschieden sind und für Alkylreste mit 1 bis 6 Kohlenstoffatomen in gerader oder verzweigter Kette stehen, sind.

11. Verfahren nach einem der Ansprüche 6 bis 10, für das das Molverhältnis von starker organischer Base mit einem pKa-Wert von mehr als 20 oder Natriumimidazolat zu quartärem Ammoniumsalz von Heparinbenzylester zwischen 0,2 und 5 liegt.

12. Verfahren nach einem der Ansprüche 6 bis 11, für das das quartäre Ammoniumsalz von Heparinbenzylester einen Veresterungsgrad zwischen 50 und 100% aufweist.

13. Verfahren nach einem der Ansprüche 6 bis 12, für das die Umwandlung des depolymerisierten quartären Ammoniumsalzes von Heparinbenzylester in ein Natriumsalz durch Behandeln des Reaktionsmediums mit einer alkoholischen Lösung von Natriumacetat erfolgt.

14. Verfahren nach einem der Ansprüche 6 bis 13, für das die Verseifung mit Hilfe eines Alkalimetallhydroxids erfolgt.

15. Verfahren nach einem der Ansprüche 6 bis 14, für das die Reinigung mit Hilfe von Wasserstoffperoxid erfolgt.

16. Gemische gemäß einem der Ansprüche 1 bis 5 als Arzneimittel.

17. Gemische gemäß einem der Ansprüche 1 bis 5 als antithrombotische Arzneimittel.

18. Pharmazeutische Zusammensetzungen, die als Wirkstoff ein Gemisch nach einem der Ansprüche 1 bis 5 enthalten.

## Claims

1. Mixtures of sulphated polysaccharides possessing the general structure of the constituent polysaccharides of heparin and exhibiting the following characteristics:
- they have a mean molecular weight of 1500 to 3000 daltons, an anti-Xa activity of 125 to 150 IU/mg, an anti-IIa activity of 0 to 10 IU/mg and an anti-Xa activity/anti-IIa activity ratio greater than 10,
- the constituent polysaccharides of the mixtures contain 2 to 26 saccharide units and have a 4,5-unsaturated glucuronic acid 2-O-sulphate unit at one of their ends,
in the form of an alkali or alkaline-earth metal salt.

2. Mixtures according to Claim 1, **characterized in that** the anti-Xa activity is between 140 and 150 IU/mg and the mean molecular weight is between 2000 and 3000 daltons.

3. Mixtures according to Claim 1 or 2, in the form of the sodium, potassium, calcium or magnesium salt.

4. Mixtures according to any one of Claims 1 to 3, having an anti-IIa activity of 0 to 5 IU/mg.

5. Mixtures according to one of Claims 1 to 4, having an anti-Xa activity/anti-IIa activity ratio greater than 25.

6. Method of preparing the mixtures of Claim 1, **characterized in that**:
- a quaternary ammonium salt of the benzyl ester of heparin is depolymerized in an organic medium by means:
• of a strong organic base with a pka greater than 20 chosen from 2-tert-butylimino-2-diethylamino-1,3-dimethylperhydro-1,3,2-diaza-phosphorine, the bases of the family of guanidine and phosphazenes,
• or of sodium imidazolate,
- the quaternary ammonium salt of the benzyl ester of the depolymerized heparin is converted to a sodium salt,
- the ester is saponified and the product is optionally purified.

7. Method according to Claim 6, for which the quaternary ammonium salt of the benzyl ester of heparin is the benzethonium, cetylpyridinium or cetyltrimethylammonium salt.

8. Method according to Claim 6, for which the bases of the guanidine family are those of formula: 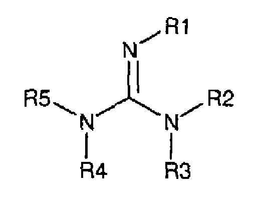 in which R₁ is hydrogen or alkyl, R₂, R₃, R₄ and R₅, which are identical or different, each represent an alkyl radical, the alkyl radicals having 1 to 6 carbon atoms in the form of a straight or branched chain.

9. Method according to Claim 8, for which R₁ is hydrogen and R₂, R₃, R₄ and R₅ are methyl radicals.

10. Method according to Claim 6, for which the bases of the phosphazene family are those of formula: 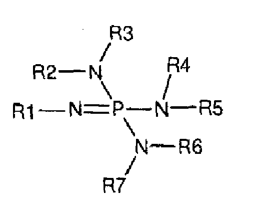 in which the radicals R₁ to R₇ are identical or different and represent alkyl radicals containing 1 to 6 carbon atoms in the form of a straight or branched chain.

11. Method according to one of Claims 6 to 10, for which the strong organic base with a pka greater than 20 or sodium imidazolate/quaternary ammonium salt of the benzyl ester of heparin mol ratio is between 0.2 and 5.

12. Method according to one of Claims 6 to 11, for which the quaternary ammonium salt of the benzyl ester of heparin has a degree of esterification of between 50 and 100%.

13. Method according to one of Claims 6 to 12, for which the conversion of the quaternary ammonium salt of the benzyl ester of the depolymerized heparin to a sodium salt is carried out by treating the reaction medium with an alcoholic solution of sodium acetate.

14. Method according to one of Claims 6 to 13, for which the saponification is carried out by means of an alkali metal hydroxide.

15. Method according to one of Claims 6 to 14, for which the purification is carried out by means of hydrogen peroxide.

16. Mixtures as defined in any one of Claims 1 to 5, as medicaments.

17. Mixtures as defined in any one of Claims 1 to 5, as antithrombotic medicaments.

18. Pharmaceutical compositions comprising, as active ingredient, a mixture according to one of Claims 1 to 5.
